# EUROPEAN PATENT APPLICATION

(11) **EP 3 023 446 A1**
(43) Date of publication of application: **25.05.2016**
(21) Application number: 14193691.4
(22) Date of filing: 18.11.2014
(51) Int. Cl.: C08G 18/48, C08G 18/73, C08G 18/08, C08G 18/22, C08G 18/28, C11D 3/37, A61K 8/87

(54) **Continuous high-throughput process for the preparation of polyurethanes**

(71) Applicant: BASF SE, 67056 Ludwigshafen am Rhein (DE)
(72) Inventor: Fernandez-Ramirez, Gimmy Alex, 67063 Ludwigshafen (DE); Crone, Benedikt, 68165 Mannheim (DE); Stadler, Daniel, 200040 Shanghai (CN); Böttcher, Axel, 41564 Kaarst (DE)
(74) Representative: Fitzner, Uwe

(57) **Abstract**

Continuous high-throughput process for the preparation of polyurethanes, with the use of rotor-stator reactors, the resulting polyurethanes and their uses.

## Description

Any of the documents cited herein are incorporated by reference in their entirety.

The instant invention concerns a high-throughput process for the preparation of polyurethanes.

### Background of the invention:

Polyurethanes and their preparation has been known in the art for many decades and the number of publications in this regards are countless.Yet it is still desirable to improve upon the preparation processes and thus the properties of the resulting polyurethanes.

One possible use of polyurethanes is their use as thickeners. The thickening effect relates to the specific properties of the respective polyurethanes.

From DE 28 33 762 A1 it is known to prepare polyurethanes in a two-step process in which all the educts (polyols, isocyanates and if desired further ones) are mixed together before entering the reaction zone.

From EP 1 876 195 A2 also two-step preparations of polyurethanes are known in which polyols and isocyanates are mixed together before actually entering the reaction zone.

The batch processes of the prior art for preparing polyurethanes are only feasible for low and medium melt-viscosities, some even for slightly higher melt-viscosities. However the batch processes of the prior art are not suitable to handle high or very high viscosity polyurethane-melts.

### Object of the invention:

It was an object of the instant invention provide a continuous preparation process for polyurethanes which is easier than the processes of the prior art, can be used to prepare high viscous polymers, preferably very high ones. Furthermore the instant invention's process should be applicable without the use of solvents. Moreover the preparation of the polyurethanes should be possible preferably without the use of tin compounds. The polyurethanes of the instant invention when used as thickeners should have similar costs and performances compared to thickeners based on high molecular weight, nonlinear polyacrylic acid cross-linked with polyalkenyl polyethers of the prior art while retaining good properties, especially the instant invention's polyurethanes should be tolerant towards salts. Accordingly the instant invention's process and polyurethanes should be highly economical, highly ecological and highly environmental friendly while at the same time showing good properties.

### Summary of the invention:

The object of the instant invention has been solved by the specific process of the instant invention as well as the specific polyurethanes of the instant invention, especially if prepared with the instant invention's process and the instant invention's method of using the polyurethanes, especially as thickeners.

### Definition of terms:

The following description is made for the purpose of illustrating the general principles of the present invention and is not meant to limit the inventive concepts claimed herein. Further, particular features described herein can be used in combination with other described features in each of the various possible combinations and permutations.

Unless otherwise specifically defined herein, all terms are to be given their broadest possible interpretation including meanings implied from the specification as well as meanings understood by those skilled in the art and/or as defined in dictionaries, treatises, etc.

It must also be noted that, as used in the specification and the appended claims, the singular forms "a", "an" and "the" include plural referents unless otherwise specified.

As used herein, the term "about" when combined with a value refers to plus and minus 10% of the reference value unless otherwise specified. For example, a temperature of about 50°C refers to a temperature of 50°C ± 5°C, etc.

Any indications of quantity given in the instant invention are to be considered as indications of quantity by weight, if not specified otherwise.

In the instant invention the term "room temperature" is intended to mean a temperature of 20°C; if not specified otherwise, temperature values are to be considered as degrees centigrade (°C).

In the instant invention are the given reactions or process steps are carried out at normal pressure/atmospheric pressure, that is at 1013 mbar.

In the instant invention the term "and/or" includes any and all combinations of one or more of the associated listed items.

The term average molecular weight in the instant invention means the weight average molecular weight unless otherwise stated.

In the context of the instant invention the term "monool" means alcohols having one hydroxyl group appended and the term "polyol" means alcohols having two or more hydroxyl groups appended.

As used in the instant invention, the term "solvent" encompasses both water and organic solvents.

In the context of the instant invention,unless otherwise stated or given, the term "high viscosity" means 10 to 50 Pas at 80°C and a shear rate of 100 s⁻¹ and the term "very high viscosity" means 50 to 700 Pas at 80°C and a shear rate of 100 s⁻¹ (or even higher), measured with a cone plate viscometer respectively, i.e. dynamic viscosities.

### Detailed description:

In the drawings and description that follows, like parts are marked throughout the specification and drawings with the same reference numerals, respectively. The drawing figures are not necessarily to scale. Certain features of the invention may be shown exaggerated in scale or in somewhat schematic form and some details of conventional elements may not be shown in the interest of clarity and conciseness.

One aspect of the instant invention is a continuous high-throughput process for the preparation of polyurethanes, comprising or consisting of the process steps of
0) optionally drying the necessary educts,
1) providing a polyol component comprising either a polyol or a polyol/monool mixture,
2) feeding the polyol component from 1) into a pre-reaction zone of a reactor,
3) adding an isocyanate component to the polyol component after the pre-reaction zone and at the beginning of the reaction zone of a reactor thus creating a reaction mixture,
4) optionally adding additives to the reaction mixture either
   4a) together with the isocyanate component or
   4b) after addition of the isocyanate component or
   4c) together with the isocyanate component and after addition of the isocyanate component,
5) reacting the reaction mixture at constant temperature in the reaction zone of the reactor,
6) optionally solving the resulting polyurethane in water, optionally with the aid of at least one surfactant and/or detergent,
   the reactor being a dynamic mixer and having
   i) a conical pre-reaction zone, and
   ii) a reaction zone having as mixing means
   iia1) a plurality of rotating wheels having a plurality of notches,
      or
   iia2) a rotating barrel having a plurality of notches,
   iib) a wall surrounding iia1) or iia2) having a plurality of notches,
      and
   iii) one or more feeding pipes along the reaction zone through which the isocyanate component and the optional additives can be fed
   iv) an exit area from which the reaction product can exit the reactor.

With this process it is possible to continuously produce amounts of polyurethanes of up to 10 tons per hour, preferably from one kilogram per hour to 10 tons per hour.

The instant invention's process preferably leads to polyurethanes having
I) having high structural viscosities,
II) being water soluble,
III) having water thickening properties.
   The polyurethanes prepared with the instant invention's process also
IV) have highly constant product properties.

Further, the instant invention also encompasses polyurethanes prepared by the instant invention's process.

Further, the instant invention also encompasses polyurethanes
I) having high structural viscosities,
II) being water soluble,
III) having water thickening properties.

A further important aspect of the instant invention is the use of the instant invention's polyurethanes or the polyurethanes prepared by a process according to the instant invention as associative thickeners, or in other words a method of using these polyurethanes as associative thickeners by adding them to the system to be thickened. They work especially well as thickeners in water-based, i.e., aqueous compositions.

The instant invention's process preferably is a solvent-free process.

That means that the viscosity of the melt is low enough at the working temperature so that no additional solvents are needed, preferably from 10 Pas at 80°C and a shear rate of 100 s⁻¹ to 700 Pas at 80°C and a shear rate of 100 s⁻¹.

Usually the instant invention's process is a melt process, that means the educts are provided in a molten state at temperatures above room temperature.

Useable polyols according to the instant invention are in principle any polyols known to the art in the preparation of polyurethanes that have two or more hydroxyl groups appended. It is preferable to employ polar alcohols. Preferred examples are polyethylene glycols of molecular masses from 200 to 100000 (number average molecular weight)

The usable polyols, besides having appended hydroxyl groups, can have hydrophilic sections S.

The sections S, independently of one another, may be identical or different, linear or branched.

In one embodiment, the hydrophilic sections S are of different lengths and linear.

In specific embodiments, the sections S may comprise radicals of alkylene oxides. In further embodiments, the number is in the range from 2 to 150 alkylene oxide radicals, or more specifically, in the range from 5 to 100 alkylene oxide radicals and in very specific embodiments in the range from 10 to 100 alkylene oxide radicals.

In another embodiment, the hydrophilic sections S may comprise or consist of ethylene oxide radicals. In one specific embodiment, the hydrophilic sections S comprise ethylene oxide radicals (EO units), the number of which is in the range from 2 to 150 EO units, in specific embodiments in the range from 5 to 100 EO units and in very specific embodiments in the range from 10 to 100 EO units.

In one embodiment, the sections S consist of 5 to 30, and in some embodiments, 10 to 25 EO units.

In another embodiment, the sections S consist of 30 to 100, and in some embodiments, 40 to 100 EO units.

The number of EO units per molecule of ethoxylated alcohol is also referred to as degree of ethoxylation.

The ethoxylated alcohols used can have a degree of ethoxylation which is in the range from 2 to 150 ethylene oxide radicals, and in some embodiments, in the range from 5 to 100 ethylene oxide radicals and, in specific embodiments, in the range from 10 to 100 ethylene oxide radicals.

In a further embodiment, the ethoxylated alcohols used have a degree of ethoxylation in the range from 10 to 25 ethylene oxide radicals.

In one embodiment, a branched, nonionic compound of the structural formula RO(CH₂CH₂O)ₓH, where R is a C13-alkyl radical, and in some embodiments, an iso-C₁₃-alkyl radical, and where x=3, 5, 6, 6.5, 7, 8, 10, 12, 15 or 20, and in some embodiments, x=10, is used as at least one of the alcohols used.

In one embodiment, at least one of the alcohols used is an alcohol of the general formula (4) in EP 761 780 A2, page 4

R⁴-CHR⁵-R⁶OH

or an alcohol of the general formula (5) in EP 761 780 A2, page 4

R⁷-CHR⁸-OH

where
R⁴, R⁵, R⁷ and R⁸, independently of one another, have the meaning described in EP 761 780 A2, page 4, lines 45 to 58; in some embodiments, R⁴, R⁵, R⁷ and R⁸, independently of one another, are alkyl radicals having at least 4 carbon atoms and the total number of the carbon atoms in the alcohols is at most 30, R⁶ is an alkylene radical, such as, for example, -CH₂-, -CH₂-CH₂-, -CH₂-CH(CH₃)-.

By way of example, mention may be made here of 2-decyl-1-tetradecanol as a suitable alcohol.

In one embodiment, mixtures of ethoxylated linear and ethoxylated branched long-chain alcohols, in particular mixtures of the aforementioned types, are used.

In one embodiment, the hydrophilic sections S comprise mixtures of EO and PO units.

The sections S can likewise comprise longer-chain alkylene oxide radicals, with the proviso that the sections S must be hydrophilic overall. The hydrophilicity can be controlled for example via the fraction of EO units in the sections S.

The hydophilic sections can be essentially linear polyether radicals, for example polyalkylene oxides. Theses can in one embodiment be radicals of polyetherdiols, especially of polyether glycoles.

The essentially linear polyether radicals can in one embodiment have number average molecular weights of between 1500 and 20000 g/mol. In another embodiment they can have number average molecular weights of between 1500 and 12000 g/mol.

In just another embodiment they can have number average molecular weights of between 4000 and 9000 g/mol. One specific example would have a number average molecular weight of 6000 g/mol or greater.

Preferred polyols to be used in the instant invention are: polyethylene or polypropylene glycols having average molecular weights of from 250 to 15000 g/mol.

Especially preferred are polyethylene glycols having higher viscosities, of more than 75 centistokes [75 * 10⁻⁶m²s⁻¹] at 99°C, being those with average molecular weights of more than 3000 g/mol, especially from 3000 to 12000 g/mol. Especially preferred are polyethylene glycols selected from the group consisting of polyethylene glycols having average molecular weights of from 5400 to 6600 g/mol, from 7000 to 9000 g/mol and mixtures thereof, one example would be a polyethylene glycol having number average molecular weight of about 9000 g/mol and a kinematic viscosity of about 900 centistokes [900 * 10⁻⁶m²s⁻¹) at 99°C.

Useable monools according to the instant invention are essentially all monols, preferred are C₄-C₃₀ alcohols, which can be alkoxylated.

Suitable alcohols are, for example, the alkoxylated linear alcohols from natural sources or from the Ziegler build-up reaction of ethylene in the presence of aluminum alkyl catalysts. Examples of suitable alcohols are C₆-C₃₀-alcohols, in particular C₁₂-C₃₀-alcohols. Particularly specific alcohols which may be mentioned here are: n-dodecanol, n-tetradecanol, n-hexadecanol, n-octadecanol, n-eicosanol, n-docosanol, n-tetracosanol, n-hexacosanol, n-octacosanol and/or n-triacontanol, and also mixtures of the aforementioned alcohols, oxo alcohols such as, for example, isoheptanol, isooctanol, isononanol, isodecanol, isoundecanol, isotridecanol, alcohols which are branched in the 2 position; these are the Guerbet alcohols known to the person skilled in the art which are accessible by dimerization of primary alcohols via the so-called Guerbet reaction, alcohols which are obtained by the Friedel-Crafts alkylation with oligomerized olefins and which then comprise an aromatic ring as well as a saturated hydrocarbon radical. Particularly specific embodiments of alcohols which may be mentioned here are: isooctylphenol and isononylphenol, alcohols of the general formula (4) in EP 761 780 A2, page 4

R⁴-CHR⁵-R⁶OH

or alcohols of the general formula (5) in EP 761 780 A2, page 4

R⁷-CHR⁸-OH

where
R⁴, R⁵, R⁷ and R⁸, independently of one another, have the meaning described in EP 761 780 A2, page 4, lines 45 to 58; more specifically, R⁴, R⁵, R⁷ and R⁸, independently of one another, are alkyl radicals having at least 4 carbon atoms and the total number of the carbon atoms in the alcohols is at most 30, R⁶ is an alkylene radical, such as, for example, -CH₂-, -CH₂-CH₂-, -CH₂-CH(CH₃)-.

By way of example, mention may be made here of 2-decyl-1-tetradecanol as suitable alcohol.

In one embodiment, mixtures of ethoxylated C₁₆-C₁₈-fatty alcohols can be used. In a further embodiment, ethoxylated iso-C₁₃-oxo alcohols or mixtures thereof can be used.

In a further embodiment, mixtures comprising ethoxylated C₁₆-C₁₈-fatty alcohols and ethoxylated iso-C₁₃-oxo alcohols can be used.

In a further embodiment, the above-described alcohols of the general formula (4) or (5) in EP 761 780 A2, page 4, can be used in their ethoxylated form

In one preferred embodiment monools to be used in the instant invention are C₁₀-C₁₈ alcohols with 4 to 100 EO units.

Especially preferred are alkylpolyethylene glycol ethers either alone or in admixture with ethoxylated fatty acid alcohols.

These alkylpolyethylene glycol ethers are preferably made from a linear, saturated C₁₆C₁₈ fatty alcohol and conform to the formula RO(CH₂CH₂O)ₓH, with R = linear, saturated C₁₆C₁₈ fatty alcohol and x = from 11 to 80, preferred 11, 13, 18, 25, 50 or 80, wherein the number in the alphanumeric code indicates the nominal degree of ethoxylation, from which the most preferred one is with an ethoxylation degree of 11.

These ethoxylated fatty acid alcohols preferably are made from a saturated iso-C₁₃ alcohol and conform to the structural formula RO(CH₂CH₂O)ₓH, with R = iso-C₁₃H₂₇ and x = from 3 to 20, preferred 3, 5, 6, 6.5, 7, 8, 10, 12, 15 or 20, from which the most preferred one is with an ethoxylation degree of 10.

One preferred mixture of monools consists of such an C₁₆C₁₈alkylpolyethylene glycol ether with an ethoxylation degree of 11 and an ethoxylated fatty acid iso-C₁₃H₂₇ alcohol with an ethoxylation degree of 10.

Useable isocyanates according to the instant invention generally are represented by the general formula R-(NCO)ₘ, where R is an m-functional organic radical with m being 2 or more. Useable isocyanates are for example (but not only) aliphatic, cycloaliphatic, aromatic or heterocyclic organic di- and poly-isocyanates having at least two isocyanate groups per molecule as well as isocyanurates and also mixtures thereof.

The organic polyisocyanate can for example be ethylene diisocyanate, 1,2-diisocyanatopropane, 1,3-diisocyanatopropane, 1,4-butylene diisocyanate, lysine diisocyanate, 1,4-methylene bis (cyclohexyl isocyanate), isophorone diisocyanate (= 5-isocyanato-1-isocyanatomethyl-1,3,3-trimethyl-cyclohexane), 5-isocyanato-1-(2-isocyanatoeth-1-yl)-1,3,3-trimethyl-cyclohexane, 5-isocyanato-1-(3-isocyanatoprop-1-yl)-1,3,3-trimethyl-cyclohexane, 5-isocyanato-(4-isocyanatobut-1-yl)-1,3,3-trimethyl-cyclohexane, 1-isocyanato-2-(3-isocyanatoprop-1-yl)-cyclohexane, 1-isocyanato-2-(3-isocyanatoeth-1-yl)-cyclohexane, 1-isocyanato-2-(4-isocyanatobut-1-yl)-cyclohexane, 1,2-diisocyanato cyclobutane, 1,3-diisocyanato cyclobutane, 1,2-diisocyanato cyclopentane, 1,3-diisocyanato cyclopentane, 1,2-diisocyanato cyclohexane, 1,3-diisocyanato cyclohexane, 1,4-diisocyanato cyclohexane, dicyclohexyl methane-2,4'-diisocyanate, trimethylene diisocyanate, tetramethylene diisocyanate, pentamethylene diisocyanate, ethylethylene diisocyanate, trimethylhexane diisocyanate, heptanemethylene diisocyanate isocyanates derived from dimeric fatty acids, 2-heptyl-3,4-bis(9-isocyanatononyl)-1-pentyl-cyclohexane, 1,2-, 1,4- or 1,3-bis(isocyanatomethyl)cyclohexane, 1,2-, 1,4- or 1,3-bis(2-isocyanatoeth-1-yl)cyclohexane, 1,3-bis(3-isocyanatoprop-1-yl)cyclohexane, 1,2-, 1,4- or 1,3-bis(4-isocyanatobut-1-yl)cyclohexane, xylene diisocyanate, toluene diisocyanate, isocyanurates of toluene diisocyanate, diphenylmethane 4,4'-diisocyanate, isocyanurates of diphenylmethane 4,4'-diisocyanate, methylenebis-4,4'-isocyanatocyclohexane, isocyanurates of isophorone diisocyanate, hexamethylene diisocyanate, isocyanurates of hexamethylene diisocyanate, 1,4-cyclohexane diisocyanate, p-phenylene diisocyanate, triphenylmethane 4,4',"-triisocyanate, tetramethyl xylylene diisocyanate, tolylene diisocyanate, bisphenylene diisocynate, naphthylene diisocyanate, diphenylmethane diisocyanate and metaxylene diisocyanate.

Preferred isocyanates to be used in the instant invention are selected from the group consisting of 1,6-hexamethylene diisocyanate, toluylene diisocyanate, isophorone diisocyanate, 4,4'-methylene diphenyl diisocyanate and mixtures thereof.

The most preferred isocyanate is 1,6-hexamethylene diisocyanate.

Additionally, it may sometimes be desired to add monoisocyanates in minor amounts, in one embodiment up to a maximum of 0.1 equivalents. Examples for those are phenyl isocyanate, cyclohexyl isocyanate, stearyl isocyanate, vinyl isocyanate, (meth)acryloyl isocyanate and/or 1-(1-isocyanato-1-methylethyl)-3-(1-methylethenyl)-benzene.

Polyols, monools and isocyanates together form a composition that is also referred to as reactive composition in the instant invention.

In the instant invention the ratio (mol:mol) of the employed alcohols to diisocyanates can be in the range of 1:1.1 to 1: 1.9. Preferably the ratio is in the range of 1:1.1 to 1:1.8. More preferably the ratio is in the range of 1:1.1 to 1:1.75. Most preferably the ratio is in the range of 1:1.2 to 1:1.75.

Of course, the ratio can also be 1:x with x being equal or greater than 1.3, preferably equal or greater than 1.5

In one embodiment, in addition to the mentioned ranges of diols, especially polyetherdiols, to diisocyanates the ratio of polyetherdiols to ethoxylated fatty alcohols is chosen such that the ratio of employed polyetherdiols to employed fatty alcohols is in the range of from 5:1 to 1:2. Preferably this ratio is in the range of from 2:1 to 1:1.8, more preferred in the range of from 1:1 to 1:1.6 and most preferably at about 1:1.5.

Especially preferred is a ratio (mol/mol) of polyetherdiols to diisocyanates to ethoxylated fatty alcohols of 1:1.75:1.5.

Useable additives according to the instant invention are for example (but not only) dispersing agents and/or stabilisers, surface active agents, conserving agents, anti foam additives, fragrances, wetting agents, thickeners, colorants, pigments, plasticisers, humectants and/or further polymers, though in one embodiment further polymers are not encompassed by the term additives in the context of the instant invention.

Emulsifiers as well as mixtures of emulsifiers with surface active agents are also considered to be surface active agents within the scope of the instant invention. As salt within the context of the instant invention salts as well as salt-like structures, also with low pks-values and mixtures thereof are encompassed. The instant invention's polyurethanes can in one preferred embodiment be used to prepare compositions that contain at least 0.05 percent by weight of salt and/or at least 0.5 percent by weight of surface active agents, preferably at least 0.1 percent by weight of salt and/or at least 1 percent by weight of surface active agents.

It is possible to use catalysts in the reaction of polyol component and isocyanate component according to the instant invention.

Examples of useable catalysts are aminic compounds, carboxylic acids or organometallic compounds.

Examples of amine compounds that may be used as a catalyst are: triethylamine, tributylamine, dimethylbenzylamine, dicyclohexylmethylamine, dimethylcyclohexylamine, N,N,N',N'-tetramethyldiaminodiethyl ether, bis(dimethylaminopropyl)urea, N-methyl-and N-ethylmorpholine, N,N'-dimporpholinodiethyl ether, N-cyclohexylmorpholine, N,N,N',N'-tetramethylethylenediamine, N,N,N',N'-tetramethylbutanediamine, N,N, N',N'-tetramethylhexane-1,6-diamine, pentamethyldiethylenetriamine, dimethylpiperazine, N-dimethylaminoethylpiperidine, 1,2-dimethylimidazole, N-hydroxypropylimidazole, 1-azabicyclo[2.2.0]octane, and 1,4-diazabicyclo[2.2.2] octane. Amine compounds also include alkanolamine compounds, such as triethanolamine, triisopropanolamine, N-methyl-and N-ethyldiethanolamine, dimethylaminoethanol, 2(N,N-dimethylaminoethoxy)ethanol, N,N',N-tri(dialkylaminoalkyl) hexahydrotriazines, such as N,N',N-tris(dimethylaminopropyl)-s-hexahydrotriazine.

Useable are also iron(II) chloride, zinc chloride, lead octoate and tin salts, such as tin dioctoate, tin diethylhexoate, dibuthyltin dilaurate and dibutyldilauryltin mercaptide, 2,3-dimethyl-3,4,5,6-tetrahydropyrimidine, tetraalkylammonium hydroxides, such as tetramethylammonium hydroxide, alkali metal hydroxides, such as sodium hydroxide, alkali metal alkoxides, such as sodium methoxide and potassium isopropoxide, and/or alkali metal salts of long-chain fatty acids having 10 to 20 carbon atoms and, optionally, pendant OH groups. Further suitable catalysts include Ti compounds, such as Ti(IV)-O-alkyl compounds with alkyl groups, such as methyl, ethyl, n-propyl, isopropyl, n-butyl, isobutyl, tert-butyl, n-pentyl, 2-pentyl, 3-pentyl, ethyl, n-propyl, isopropyl, n-butyl, isobutyl, tert-butyl and Ti(IV) butoxide. Suitable organometallic compounds of tin, lead, iron, titanium, bismuth or zirconium may be used as catalyst, and include tetraisopropyl titanate, lead phenylethyldithiocarbamate, tin(II) salts of carboxylic acids, such as tin(II) acetate, ethylhexoate and diethylhexoate, for example. Dialkyltin(IV) carboxylates, tin oxides, tin sulphides and tin thiolates Specific compounds include the following: bis(tributyltin)oxide, bis(trioctyltin)oxide, dibutyltin and dioctyltin bis(2- ethylhexyl thiolate), dibutyltin and dioctyltin didodecylthiolate, bis(-methoxycarbonylethyl)tin didodecylthiolate, bis(-acetylethyl)tin bis(2-ethylhexylthiolate), dibutyltin and dioctyltin didodecylthiolate, butyltin and octyltin tris(thioglycolic acid-2-ethylhexoate), dibutyl- and dioctyltin- bis(thioglycolic acid 2-ethylhexoate), tributyl-and trioctyltin(thioglycolic acid 2-ethylhexoate), butyltin and octyltin tris(thioethylene glycol 2-ethylhexoate), dibutyltin and dioctyltin bis(thioethylene glycol-2-ethylhexoate), tributyltin and trioctyltin (thioethylene glycol 2-ethylhexoate), bis(-methoxycarbonylethyl)tin bis(thioethylene glycol 2-ethylhexoate), bis(-methoxycarbonylethyl)tin bis(thioglycolic acid 2-ethylhexoate), bis(-acetylethyl)tin bis(thioethylene glycol 2-ethylhexoate) and bis(-acetylethyl)tin bis(thioglycolic acid 2-ethylhexoate). Organobismuth compounds are, in particular, bismuth carboxylates of mono or dicarboxylic acids in which the carboxylic acids have 2 to 20 carbon atoms. Specific acids that may be mentioned include the following: adipic acid, maleic acid, fumaric acid, malonic acid, succinic acid, pimelic acid, terephthalic acid, phenylacetic acid, benzoic acid, acetic acid, propionic acid, 2- ethylhexanoic, caprylic, capric, lauric, myristic, palmitic and stearic acid. Acids also include the following: butyric acid, caproic acid, caprylic acid, capric acid, lauric acid, myristic acid, palmitic acid, stearic acid, arachidic acid, isobutyric acid and 2- ethylhexanoic acid. It is also possible to use mixtures of bismuth carboxylates with other metal carboxylates, such as tin carboxylates.

Preferably, the employed catalysts are not tin-compounds.

It is preferred in the instant invention to use zinc or titanium compounds as catalysts, especially preferred is zinc neodecanoate.

If catalysts are used, their amount, relative to the reactive composition, is 0.1 to 10 % by weight, preferably 0.2 to 2 % by weight, especially preferred between 0.5 and 0.7% by weight.

Of course mixtures of two or more of the catalysts can be employed according to the instant invention. It is preferred, though, to use only one catalyst compound at a time.

In the instant invention's process, the reaction mixture is under constant high shear forces in the reaction zone, preferably only in the reaction zone but neither in the pre-reaction zone nor the exit area, wherein as high shear forces such that have a range of between 1000 s⁻¹ and 18000 s⁻¹, preferably between 1500 s⁻¹ and 10000 s⁻¹, are to be understood.

To be used as reactors within the context of the instant invention are high shear mixers, preferably dynamic mixers. These produce higher shear forces than stationary mixers.

The dynamic mixers to be used in the instant invention are preferably of the rotor-stator-type and feature a conical entrance, also called pre-reaction zone in the instant invention, before the mixing zone, also called reaction zone in the instant invention.

Due to the conical entrance area, only a slight pressure drop arises when the polyol/monool component enters the reactor.

Similarly, the product withdrawal area/line, also called exit zone, can in one embodiment be conical. In another embodiment it can be pipe-like or formed as a line. In general there is no limitation to the exact shape of the exit area as long as the withdrawal area is shaped such that no product jams occur, i.e., no pressure drop or increase due to its form occurs.

Preferred mixers have 12 distinct stages and are rotated with a speed of between 100 and 6000, preferably 1300 to 2500 turns per minute.

Preferably the residence time of the reaction mixture in the reaction zone is between 10 and 150 s, preferably between 10 and 100 s, especially preferred between 10 and 70 s, that residence time being a quotient of the volume of the mixing chamber and the volume current:
For example volume reaction zone = 22 ml, volume current = 2348 ml/h leads to residence time (tau) = 34 s.

An example of a commercially available reactor is a Megatron MT FM 12-50 from the company Kinematica.

In the instant invention the mean temperature of the reaction zone between monomer input and product output is kept constant.

Of course, due to practical reasons slight variations of the temperature are acceptable.

Typically, a temperature difference of three degrees Celsius is established and acceptable.

Preferably the temperature is between 20 and 250°C depending on the educts and the desired product. More preferably the temperature is between 60 and 150°C, most preferably 70 to 95°C. One especially preferred range is 75 to 90°C.

The temperatures in the entrance area and the exit area can, respectively and independent of each other, be the same as in the reaction zone or different.

It is preferred in the instant invention that the educts to be employed in the instant invention's process are water-free.

To that end when they are not provided in dry form already, it is preferred to dry them either batch-wise or continuously, particularly preferably continuously, in step 0) of the instant invention's process.

With the optional process step 6) the instant invention directly leads to aqueous thickener/thickened compositions containing the specific polyurethanes of the instant invention/ the polyurethanes prepared according to the instant invention.

Accordingly, the preferred polyurethanes of the instant invention or prepared with the instant invention's process are hydrophobically modified ethoxylated urethanes (HEUR), made from polyol, diisocyanate, hydrophobic alcohol.

The instant invention's polyurethanes have, inter alia, the following advantages. One advantage is their ability to modify the rheological properties of a composition containing these polyurethanes. The term modify in this context is to be understood to generally mean the change of deforming and flowing properties of matter. One of the most important rheological properties is viscosity. Under modification of rheological properties especially the increase of the viscosity of liquids is understood, which often is named "thickening". Such an increase in viscosity can get as high as to the formation of gels or solids. The instant invention's polyurethanes lead to an increase in the dynamic viscosity of water-containing compositions. Preferred are polyurethanes whose 10 percent by weight aqueous dispersions show a dynamic viscosity at a shear rate of 100s⁻¹ of at least 100 mPas, preferably 1000 mPas and more preferably of at least 10000 mPas.

The aqueous dispersions of the instant invention's polyurethanes may show newtonian or structure viscous behaviour, mostly they show strong structure viscous behaviour.

Structure viscous dispersions which contain the instant invention's polyurethanes preferably show dynamic viscosities of at least 1000 mPas, preferably 10000 mPas (10 percent by weight aqueous dispersions at a shear rate of 100s⁻¹).

Another advantage of the instant invention's polyurethanes and the instant invention's process for their preparation is the use of zinc- and/or titanium-containing catalysts in the preparation of the polyurethanes. Especially in the field of cosmetic appliances the prior art's preparations based on tin are no longer desired as the resulting products and the resulting cosmetic preparations still can contain tin or tin-compounds. Zinc-containing cosmetic preparations are accepted, in which also zinc can impose added advantages due to its antibacterial and anti-inflammatory properties.

Due to their high tolerance towards salt and simultaneously high amounts of surface active agents, the instant invention's polyurethanes can advantageously be used in home-care-preparations, for example as thickeners in liquid cleaners.

Furthermore the instant invention's polyurethanes are very well suited as rheology modifying agents for hydrogen peroxide-containing preparations.

The instant invention's polyurethanes or the polyurethanes prepared according to the instant invention can advantageously be used for preparations that are oil-in-water-emulsions, also if they contain additional salt.

The instant invention's preferred polyurethanes or the preferred polyurethanes prepared according to the instant invention's process have high salt tolerance, are stable upon pH-change and have a high temperature sensitivity.

An additional advantage of the instant invention is that with constant flow rate through the reactor by varying the rotational speed of the rotor of the rotor-stator reactor the exact distribution of the molar weights of the resulting polymers can be adjusted.

The instant invention's process has some considerable advantages in that it provides very high control over the resulting polyurethanes in that by varying the residence time of the compounds in the reaction zone on the one hand and by the applied shear forces in the form of the mixer's revolutions per minute on the other hand control is possible.

Accordingly, with the instant invention it has surprisingly been found a continuous high-throughput solvent free process for the preparation of polyurethanes that leads to polyurethanes having good property profiles.

One very specific embodiment of the instant invention is a continuous high-throughput process for the solvent-free preparation of highly structure viscous polyurethane associative thickeners, comprising or consisting of the process steps of
0) optionally drying the necessary educts,
1) providing a polyol component comprising a mixture of
   one or more linear poylethylenglycols having a number average molecular weight in the range of 4000 to 8000 g/mol, and
   one or more non-ionic ethoxylated fatty alcohols prepared from C₁₁ to C₂₀ alcohols, having average degrees of ethoxylation of 8 to 14, and
   a catalyst selected from the group consisting of zinc compounds, titanium compounds or mixtures thereof
2) feeding the polyol component from 1) into a pre-reaction zone of a reactor at a temperature in the range of 70 to 100°C,
3) adding an isocyanate component selected from the group consisting of 1,6-hexamethylene diisocyanate, toluylene diisocyanate and mixtures thereof to the polyol component after the pre-reaction zone and at the beginning of the reaction zone of the reactor thus creating a reaction mixture,
4) optionally adding additives to the reaction mixture either
   4a) together with the isocyanate component or
   4b) after addition of the isocyanate component or
   4c) together with the isocyanate component and after addition of the isocyanate component,
5) reacting the reaction mixture at constant temperature of between 80 to 100°C in the reaction zone of the reactor for a time of between 10 and 100 s, preferably between 10 and 70 s,
6) optionally solving the resulting polyurethane after its exit from the reactor in water, optionally with the aid of at least one surfactant and/or detergent,
   the reactor being a dynamic mixer of the rotor-stator type and having
   i) a conical pre-reaction zone, and
   ii) a reaction zone having as mixing means
      iia1) a plurality of rotating wheels having a plurality of notches, or
      iia2) a rotating barrel having a plurality of notches,
         and
      iib) a wall surrounding iia1) or iia2) having a plurality of notches, and
   iii) one or more feeding pipes along the reaction zone through which the isocyanate component and the optional additives can be fed
   iv) an exit area from which the reaction product can exit the reactor, wherein the rotational speed of the reactor is between 100 and 6000, preferably 1300 and 2500 turns per minute.

With the instant invention's process it is possible to increase the preparation efficiency manifold compared to the prior art: whereas similar (but not the same) polyurethanes in prior art's preparations via batch polymerisation in solvent take several hours and comparable batch polymerisations in the melt (solvent free) still take up to an hour, the instant invention's process can achieve the results in about one or two minutes.

With he instant invention's process it is possible to prepare polyurethanes having similar (but not the same) molecular weight distributions than those of the prior art and at the same time the application properties of the instant invention's polyurethanes/the polyurethanes prepared with the instant invention's process show better performance.

The various embodiments of the instant invention, including those of the dependent claims, can be combined with each other in any desired manner.

### Description of the drawings:

Figures 1 to 3 show the molecular weight distributions of the polyurethanes prepared in the examples 1 to 3.

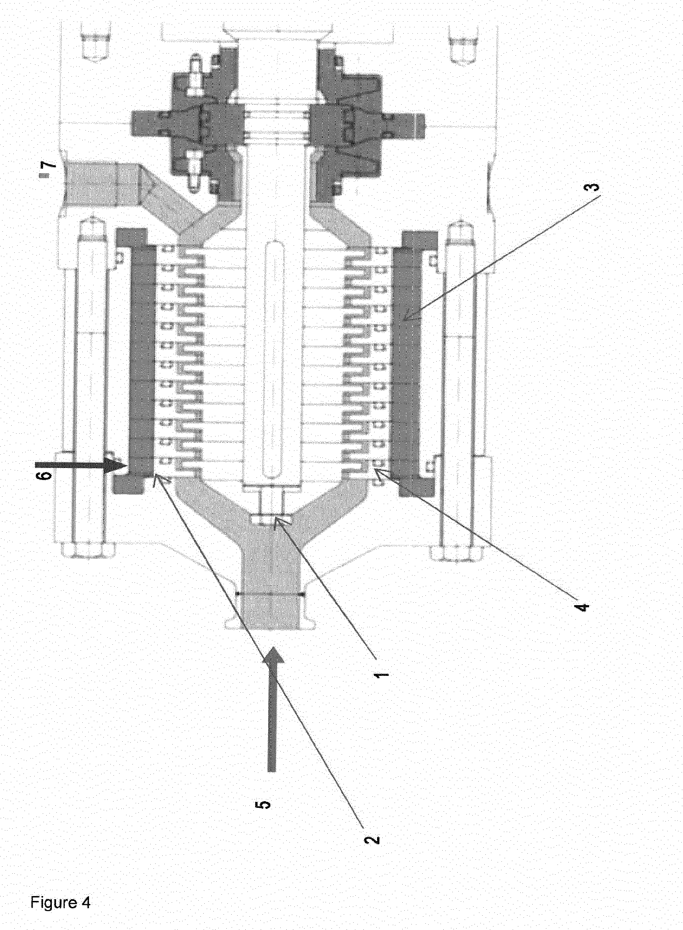

Figure 4 shows an example of the structure of a particular preferred reactor to be used in the instant invention, wherein the numerals have the following meanings:
   - 1: rotor
   - 2: stator
   - 3: double jacket
   - 4: mixing stages, 12 in this example, which form the reaction zone
   - 5: monomer (alcohols) feeding pipe/pre-reaction zone
   - 6: isocyanate feeding pipe
   - 7: product withdrawal line/exit area

### Examples:

The invention will now be explained by way of the following non-limiting examples.

In these examples the molecular weights were determined by gel permeation chromatography (GPC using a refractometer as detector). The standard used was polymethyl methacrylate (PMMA). The solvents used were N,N-dimethylacetamide (DMAc) or tetrahydrofurane (THF), if not stated otherwise.

Further, in the examples the dynamic viscosities were measured as follows:
The dynamic viscosities of the instant invention's polyurethanes in aqueous dispersion were measured in the form of a 10 percent by weight dispersion at 23°C. In the examples the dynamic viscosities were measured at shear rates of 100 s⁻¹ and 350 s⁻¹. These two values allow a statement whether the invention's polyurethanes in aqueous dispersion show structure viscous or newtonian thickening behaviour
For measuring of the dynamic viscosities according to DIN 53019 a rotational viscosimeter Physica MCR 301 with cone/plate system CP25-1 was used.

### Example 1 - solvent-free preparation of a urethane:

The reaction was done in a 12-stage dynamic mixer (Megatron MT FM 12-50). The mixer was fitted with an oil-heated double jacket. In the first stage a mixture of 2035 g/h of a linear poylethylenglykol having a number average molecular weight of 6000 g/mol, 14 g/h of zinc neodecanoate, 163 g/h non-ionic ethoxylated fatty alcohol prepared from a saturated iso-C₁₃ alcohol and an average degree of ethoxylation of 10 and 188 g/h of a non-ionic ethoxylated fatty alcohol mixture, prepared from a C₁₆/C₁₈ alcohol mixture and an average degree of ethoxylation of 11 was continuously dosed at 80°C. In the second stage of the mixer 100 g/h hexamethylene diisocyanate were added at 20°C with the aid of a HPLC pump. The mean temperature between input and output of the reaction medium was kept constant at a temperature of 80°C with the aid of the double jacket. During the reaction the number of revolutions of the mixer was kept constant at 2000 U/min. The reaction time, that is the time of the reaction mixture in the reaction zone, was 34 s (That time is the quotient between the volume of the reaction zone / volume current).

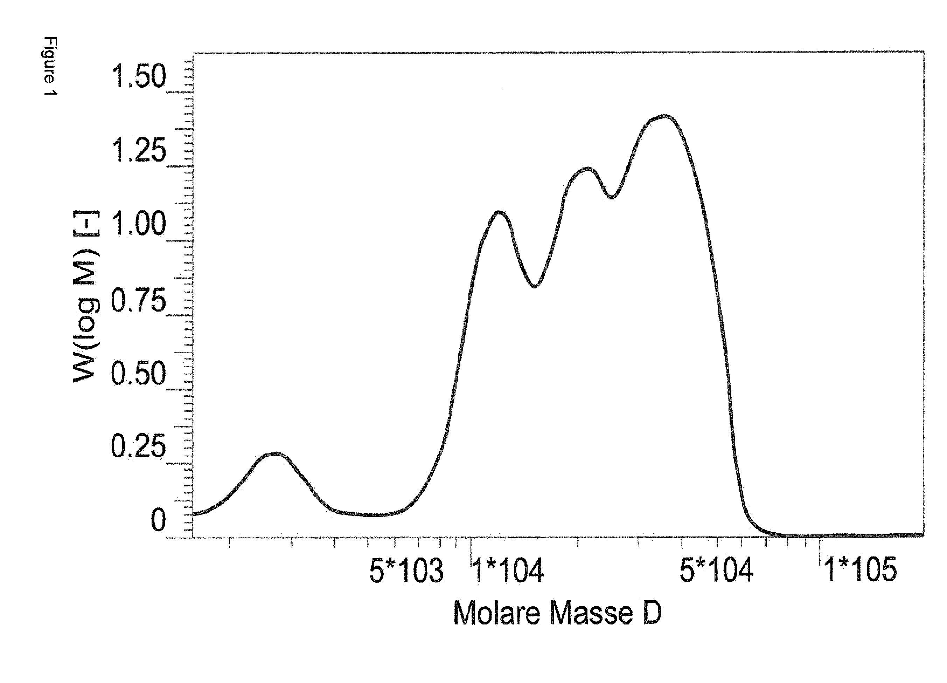

The resulting polymer 1 had the molecular weight distribution shown in figure 1. A solution of 10 percent by weight of the polymer 1 (Mₙ=1.28 10⁴ g/mol, M_{w}=2.41 10⁴ g/mol) in water showed a viscosity at 23°C of 11480 mPas (shear rate 10 1/s) and 10370 mPas (shear rate 100 1/s) and also showed strong structural viscosity, wherein both measurements were done with a rheometer Physica MCR 301 on a cone/plate system, CP25-1.

### Example 2 - solvent-free preparation of a urethane:

The reaction was done in a 12-stage dynamic mixer (Megatron MT FM 12-50). The mixer was fitted with an oil-heated double jacket. In the first stage a mixture of 1018 g/h of a linear poylethylenglykol having a number average molecular weight of 6000 g/mol, 7 g/h of zinc neodecanoate, 81 g/h non-ionic ethoxylated fatty alcohol prepared from a saturated iso-C₁₃ alcohol and an average degree of ethoxylation of 10 and 94 g/h of a non-ionic ethoxylated fatty alcohol mixture, prepared from a C₁₆/C₁₈ alcohol mixture and an average degree of ethoxylation of 11 was continuously dosed at 90°C. In the second stage of the mixer 40 g/h hexamethylene diisocyanate were added at 20°C with the aid of a HPLC pump.

The mean temperature between input and output of the reaction medium was kept constant at a temperature of 90°C with the aid of the double jacket. During the reaction the number of revolutions of the mixer was kept constant at 1500 U/min. The reaction time, that is the time of the reaction mixture in the reaction zone, was 68 s.

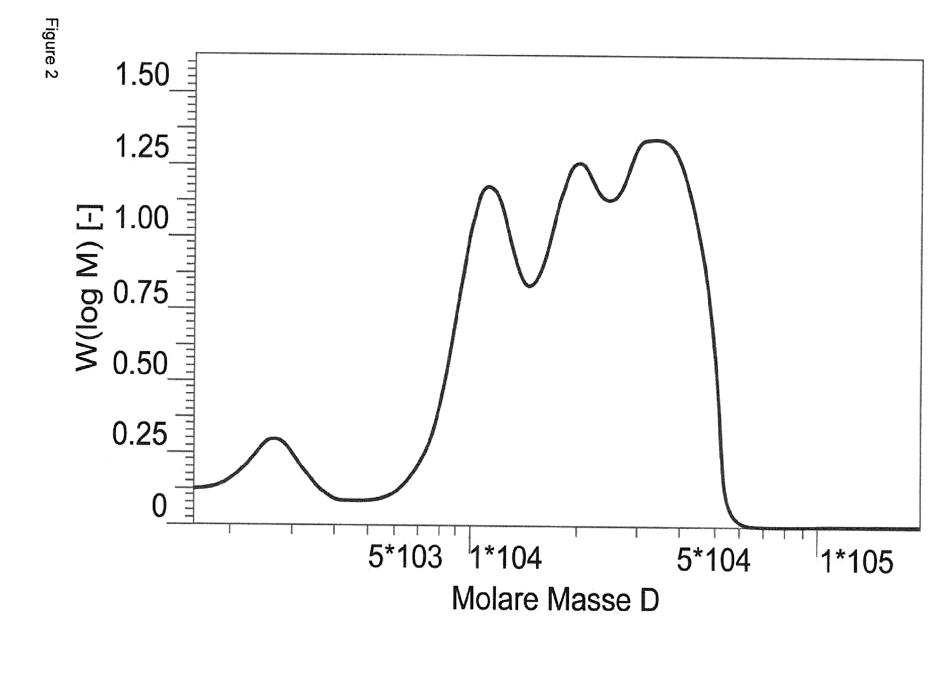

The resulting polymer 2 had the molecular weight distribution shown in figure 2. A solution of 10 percent by weight of the polymer 2 (Mₙ=1.16 10⁴ g/mol, M_{w}=2.21 10⁴ g/mol) in water showed a viscosity at 23°C of 7107 mPas (shear rate 10 1/s) and 6622 mPas (shear rate 100 1/s) and also showed strong structural viscosity, wherein both measurements were done with a rheometer Physica MCR 301 on a cone/plate system, CP25-1.

### Example 3 - solvent-free preparation of a urethane:

The reaction was done in a 12-stage dynamic mixer(Megatron MT FM 12-50).

The mixer was fitted with an oil-heated double jacket. In the first stage a mixture of 1018 g/h of a linear poylethylenglykol having a number average molecular weight of 6000 g/mol, 7 g/h of zinc neodecanoate, 81 g/h non-ionic ethoxylated fatty alcohol prepared from a saturated iso-C₁₃ alcohol and an average degree of ethoxylation of 10 and 94 g/h of a non-ionic ethoxylated fatty alcohol mixture, prepared from a C₁₆/C₁₈ alcohol mixture and an average degree of ethoxylation of 11 was continuously dosed at 90°C. In the second stage of the mixer 60 g/h hexamethylene diisocyanate were added at 20°C with the aid of a HPLC pump.

The mean temperature between input and output of the reaction medium was kept constant at a temperature of 90°C with the aid of the double jacket. During the reaction the number of revolutions of the mixer was kept constant at 1500 U/min. The reaction time, that is the time of the reaction mixture in the reaction zone, was 67 s.

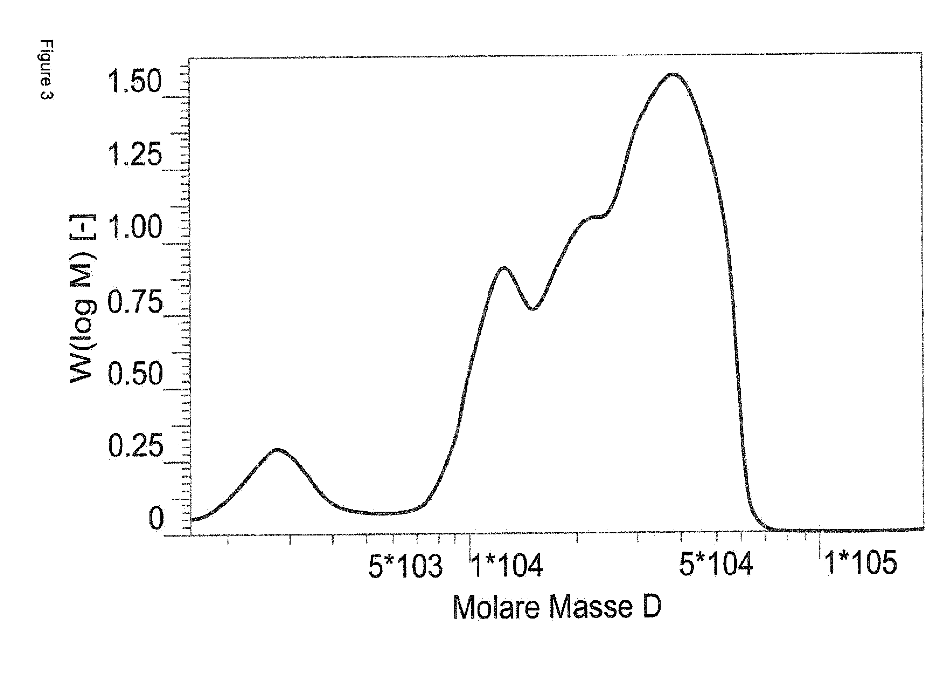

The resulting polymer 3 had the molecular weight distribution shown in figure 3. A solution of 10 percent by weight of the polymer 3 (Mₙ=1.34 10⁴ g/mol, M_{w}=2.64 10⁴ g/mol) in water showed a viscosity at 23°C of 69580 mPas (shear rate 10 1/s) and 42410 mPas (shear rate 100 1/s) and also showed strong structural viscosity, wherein both measurements were done with a rheometer Physica MCR 301 on a cone/plate system, CP25-1

All the polyurethanes of the instant invention and those that have been prepared according to the instant invention show considerably better stabilities towards salts than ASE type thickeners and and are as good as those described in WO 2009/135856. Furthermore, the polyurethane of the instant invention and those that have been prepared according to the instant invention show viscosities at cero shear rate range between 20 to 4000 Pa s at 80°C and 70°C respectively, measured with Physica MCR 301 with cone/polate system CP25-1.

## Claims

1. Continuous high-throughput process for the preparation of polyurethanes, comprising or consisting of the process steps of
0) optionally drying the necessary educts,
1) providing a polyol component comprising either a polyol or a polyoltmonool mixture,
2) feeding the polyol component from 1) into a pre-reaction zone of a reactor,
3) adding an isocyanate component to the polyol component after the pre-reaction zone and at the beginning of the reaction zone of a reactor thus creating a reaction mixture,
4) optionally adding additives to the reaction mixture either
4a) together with the isocyanate component or
4b) after addition of the isocyanate component or
4c) together with the isocyanate component and after addition of the isocyanate component,
5) reacting the reaction mixture at constant temperature in the reaction zone of the reactor,
6) optionally solving the resulting polyurethane in water, optionally with the aid of at least one surfactant and/or detergent,
the reactor being a dynamic mixer and having
i) a conical pre-reaction zone, and
ii) a reaction zone having as mixing means
iia1) a plurality of rotating wheels having a plurality of notches, or
iia2) a rotating barrel having a plurality of notches,
iib) a wall surrounding iia1) or iia2) having a plurality of notches,
and
iii) one or more feeding pipes along the reaction zone through which the isocyanate component and the optional additives can be fed
iv) an exit area from which the reaction product can exit the reactor.

2. Process according to claim 1 **characterised in that** the polyol is selected from the group consisting of polyethylene or polypropylene glycols having average molecular weights of from 250 to 15000 g/mol or mixtures thereof, preferably polyethylene glycols having average molecular weights of more than 3000 g/mol, especially from 3000 to 12000 g/mol, especially preferred polyethylene glycols selected from the group consisting of polyethylene glycols having average molecular weights of from 5400 to 6600 g/mol.

3. Process according to claim 1 or 2, **characterised in that** the monools are alkylpolyethylene glycol ethers either alone or in admixture with ethoxylated fatty acid alcohols, the alkylpolyethylene glycol ethers preferably being made from a linear, saturated C₁₆C₁₈ fatty alcohol and conforming to the formula RO(CH₂CH₂O)ₓH, with R = linear, saturated C₁₆C₁₈ fatty alcohol and x = from 11 to 80, preferred 11, 13, 18, 25, 50 or 80, wherein the number in the alphanumeric code indicates the nominal degree of ethoxylation, from which the most preferred one is with an ethoxylation degree of 11 and the ethoxylated fatty acid alcohols preferably being made from a saturated iso-C₁₃ alcohol and conform to the structural formula RO(CH₂CH₂O)ₓH, with R = iso-C₁₃H₂₇ and x = from 3 to 20, preferred 3, 5, 6, 6.5, 7, 8, 10, 12, 15 or 20, from which the most preferred one is with an ethoxylation degree of 10.

4. Process according to any one of claims 1 to 3, **characterised in that** the diisocyanate is selected from the group consisting of 1,6-hexamethylene diisocyanate, toluylene diisocyanate, isophorone diisocyanate, 4,4'-methylene diphenyl diisocyanate and mixtures thereof, preferably selected from the group consisting of 1,6-hexamethylene diisocyanate, toluylene diisocyanate and mixtures thereof.

5. Process according to any one of claims 1 to 4, **characterised in that** the employed catalysts are not tin-compounds, preferably selected from the group consisting of zinc compounds, titanium compounds or mixtures thereof, especially preferred zinc neodecanoate

6. Process according to any one of claims 1 to 5, wherein
1) the polyol component comprises a mixture of
one or more linear poylethylenglycols having a number average molecular weight in the range of 4000 to 8000 g/mol, and
one or more non-ionic ethoxylated fatty alcohols prepared from C₁₁ to C₂₀ alcohols, having average degrees of ethoxylation of 8 to 14, and
a catalyst selected from the group consisting of zinc compounds, titanium compounds or mixtures thereof
2) the polyol component from 1) is fed into a pre-reaction zone of a reactor at a temperature in the range of 70 to 100°C,
3) the isocyanate component is selected from the group consisting of 1,6-hexamethylene diisocyanate, toluylene diisocyanate and mixtures thereof,
5) the reaction mixture is reacted at constant temperature of between 80 to 100°C in the reaction zone of the reactor for a time of between 10 and 100 s, preferably between 10 and 70 s,
6) the resulting polyurethane is optionally solved after its exit from the reactor in water, optionally with the aid of at least one surfactant and/or detergent,
the reactor is a dynamic mixer of the rotor-stator type, and the rotational speed of the reactor is between 100 and 6000, preferably 1300 and 2500 turns per minute.

7. Polyurethanes prepared by a process according to any one of claims 1 to 6.

8. A method of using the polyurethanes of any one of claims 7 or 9 and/or of polyurethanes prepared according to any one of claims 1 to 6 as associative thickeners by adding them to the system to be thickened.

9. Polyurethanes **characterised in that** the polyurethanes
I) have high structural viscosities,
II) are water soluble,
III) have water thickening properties.
